Europäisches Patentamt

European Patent Office

Office européen des brevets

(19)

(11) Veröffentlichungsnummer: **0 107 159**
**B2**

(12) **NEUE EUROPÄISCHE PATENTSCHRIFT**

(45) Veröffentlichungstag der neuen Patentschrift:
25.07.90

(51) Int. Cl.⁵: **A 61 K 7/06**

(21) Anmeldenummer: **83110281.9**

(22) Anmeldetag: **15.10.83**

(54) Haarkosmetische Mittel.

(30) Priorität: **23.10.82 DE 3239296**

(43) Veröffentlichungstag der Anmeldung:
**02.05.84 Patentblatt 84/18**

(45) Bekanntmachung des Hinweises auf die
Patenterteilung:
**08.10.86 Patentblatt 84/41**

(45) Bekanntmachung des Hinweises auf die
Entscheidung über den Einspruch:
**25.07.90 Patentblatt 90/30**

(84) Benannte Vertragsstaaten:
**AT BE CH DE FR GB IT LI NL SE**

(56) Entgegenhaltungen:
**DE-A-2 421 695**

**CHEMICAL ABSTRACTS, Band 87, 1977, Seite
300, Nr. 172719d, Columbus, Ohio, US
CHEMICAL ABSTRACTS, Band 93, 1980, Seite
97, Nr. 188131r, Columbus, Ohio, US**

(73) Patentinhaber: **Henkel Kommanditgesellschaft
auf Aktien
Postfach 1100 Henkelstrasse 67
D-4000 Düsseldorf-Holthausen (DE)**

(72) Erfinder: **Hollenberg, Detlef, Dr.
Hugo-Wolf-Strasse 15
D-4010 Hilden (DE)**
Erfinder: **Cortekar, Hans-Wolfgang
Elisabeth-Strasse 38
D-4018 Langenfeld-Richrath (DE)**
Erfinder: **Giede, Karl
Schlehenweg 12
D-4010 Hilden (DE)**
Erfinder: **Höffkes, Horst, Dr.
Carlo-Schmid-Strasse 113
D-4000 Düsseldorf-Hellerhof (DE)**

**Beschreibung**

Gegenstand der Erfindung ist die Verwendung von On-Säuren in haarkosmetischen Mitteln zur Verbesserung der Struktur und Widerstandsfähigkeit der Haare, insbesondere von Haaren, die durch haarkosmetische Behandlungen wie Färben, Bleichen, Dauerwellen, nachteilig beeinflusst werden.

Durch regelmässige Behandlung mit alkalischen, stark reduzierenden oder oxidierenden Chemikalien, z.B. beim Färben, Bleichen oder Dauerwellen, erleidet das Haar eine Schädigung der Struktur. Als Folge dieser Strukturschädigung machen sich Gewichtsverlust, Brüchigkeit, erschwerte Kämmbarkeit und eine Verschlechterung von Sitz und Fülle der Friser bemerkbar. Das in dieser Weise strapazierte Haar weist darüber hinaus oft ein stumpfes und glanzloses Aussehen auf.

Um diesem Übelstand zu begegnen, hat man haarkosmetischen Präparaten bereits strukturverbessernde Wirkstoffe zugesetzt. Als Mittel zur Verbesserung der Haarstruktur wurden in erster Linie Formaldehyd und Formaldehyd abspaltende Verbindungen sowie S-Acetylsuccinanhydrid, Ammoniumvinylphosphat, Ammoniumphosponat und andere eingesetzt. Es wurde auch bereits vorgeschlagen, reduzierend wirkende Zucker zur Strukturverbesserung des Haares einzusetzen. Obwohl diese Substanzen eine gewisse Wirksamkeit zeigen, ist ihr Einsatz auf solche Zusammensetzungen beschränkt, die mit der reduzierend wirkenden Aldehydfunktion nicht in Reaktion treten. So ist der Einsatz von Glucose, z.B. in Kaltwellmitteln auf Basis von Thioglykolsäure oder in oxidierenden Medien, z.B. in Blondiermitteln oder Entwickleremulsionen für Färbecremes nicht möglich.

In der deutschen Offenlegungsschrift 24 21 695 werden Hautpflegemittel beschrieben, die Polyhydroxyalkansäuren als Haut-Feuchthaltemittel enthalten.

In der japanischen Offenlegungsschrift 55—65298 (referiert in Chemical Abstracts als Referat 93:188131r) werden Geschirrspülmittel und Shampoos beschrieben, die Gluconsäure enthalten.

In der deutschen Offenlegungsschrift Nr. 24 38 534 werden Kopfhautpflegemittel mit einem Gehalt an Uronsäuren, z.B. Glucuronsäure vorgeschlagen. Diese, ebenfalls eine unverschlossene Aldehydfunktion aufweisenden Verbindungen sind aber als Harstrukturanten praktisch unwirksam.

Es bestand daher die Aufgabe, neue Wirkstoffe aufzufinden, die in Haarbehandlungsmitteln die Struktur des Haares und die Widerstandsfähigkeit gegen den Einfluß schädigender chemischer Behandlungsmittel merklich verbessern.

Es wurde nun gefunden, daß die gestellte Aufgabe in überraschender Weise gelöst wird durch die Verwendung von On-Säuren, die von Aldohexosen oder Disacchariden mit unverschlossener Aldehydfunktion abgeleitet sind und/oder deren γ- oder δ-Lactonen als strukturverbessernde Zusätze in haarkosmetischen Mitteln.

Gegenstand der Erfindung ist daher die Verwendung von On-Säuren, die von Aldohexosen oder Disacchariden mit unverschlossener Aldehydfunktion abgeleitet sind und/oder deren γ- oder δ-Lactonen als Zusätze in Haarwässern, Haarfestigern, Haarsprays, Frisierlotionen, Frisiercremes, Frisiergelen, Wasserwellmitteln, Fönwellmitteln, Schaumtönungsmitteln. Tönungswaschmitteln, Färbemitteln, Dauerwellmitteln, Dauerwellfixierlösungen und Entwickleremulsionen für Oxidationshaarfärbepräparate zur Verbesserung der Struktur und Widerstandsfähigkeit der Haare.

Die strukturverbessernde Wirksamkeit dieser Zusätze ist überraschend, da niedermolekulare Hydroxycarbonsäure wie Glykolsäure oder Glycerin-monocarbonsäure einen vergleichbaren Effekt nicht zeigen. Auch die von Monosacchariden abgeleiteten Uronsäuren, z.B. die Gluconsäure, und die Zuckersäuren, z.B. die Schleimsäure, zeigen keine derartige Wirkung.

Die On-Säuren sind aus Aldohexosen durch vorsichtige Oxidation der Aldehydfunktion, z.B. mit Hypobromit oder mit verdünnter Salpetersäure, herstellbar. In alkalischer Lösung sind die On-Säuren als Salze beständig, die freien Säuren gehen leicht in die γ- oder δ-Lactone über, bevorzugt bilden sich die γ-Lactone.

Auch aus Disacchariden mit unverschlossener Aldehydfunktion, z.B. aus Lactose, Maltose, Gentiobiose, Cellobiose, Melibiose sind durch vorsichtige Oxidation On-Säuren zugänglich, die ebenfalls leicht in die entsprechenden γ- oder δ-Lactone übergehen.

In den haarkosmetischen Mitteln wird erfindungsgemäß bevorzugt Gluconsäure und/oder Lactobionsäure verwendet.

Die erfindungsgemäß in Haarbehandlungsmitteln verwendeten On-Säuren verbessern merklich die Struktur und Festigkeit des Haares, insbesondere aber die Widerstandsfähigkeit gegen die Schädigung, die bei regelmäßiger Anwendung von Färbemitteln, Bleichmitteln und Dauerwellmitteln auftreten können. Durch diesen Effekt wird gleichzeitig Kämmbarkeit und Glanz des Haares sowie Sitz und Fülle der Frisur günstig beeinflußt.

Besonders bevorzugt ist die Verwendung der On-Säuren in haarkosmetischen Mitteln, die starke Reduktionsmittel wie Salze von Mercaptocarbonsäuren oder von schwefliger Säure oder starke Oxidationsmittel wie Alkalibromat oder Wasserstoffperoxid enthalten, da in solchen Zubereitungen die Verringerung der Haarschädigung besonders erwünscht ist und andererseits reduzierend wirkende Zucker in diesen Präparaten nicht beständig sind. Dauerwellmittel, Dauerwellfixierlösungen und Entwickleremulsionen für Oxidationshaarfärbepräparate mit einem Gehalt an On-Säuren gehören also zu den bevorzugten Ausführungsformen der Erfindung.

Die haarkosmetischen Mittel können erfindungsgemäß On-Säuren in Mengen von 0,5 bis 10 Gew.% enthalten, bevorzugt werden Mengen von 1 bis 5 Gew.% zugesetzt. Neben diesen Wirkstoffen enthalten die Haarbehandlungsmittel die für solche Zubereitungen üblichen Hilfsmittel wie Lösungsmittel, z.B. Ethanol oder Isopropanol, oberflächenaktive Stoffe, kosmetische Ölkomponenten, polymere Filmbildner, Duftstoffe, Farbstoffe, Komplexbildner und übliche haarkosmetische Wirkstoffe wie Avivagemittel, kationische Polymere, Antischuppenwirkstoffe, Sebostatika, Vitamine, antimikrobielle Stoffe sowie die zur Haarfärbung üblichen direktziehenden Farbstoffe oder Oxidationsfarbstoffvorprodukte, die für die Entwicklung von Oxidationsfärbemitteln üblichen Oxidationsmittel, die für die Haarverformung üblichen Reduktionsmittel (z.B. Salze der Thioglykolsäure, Natriumsulfit) oder die für die Fixierung der Dauerwellen üblichen Oxidationsmittel (z.B. Natriumbromat).

Die folgenden Beispiele sollen die strukturverbessernde Wirkung der On-Säuren in haarkosmetischen Mitteln zeigen und den Gegenstand der Erfindung näher erläutern, ohne ihn jedoch hierauf zu beschränken.

Beispiele

1. Prüfung der strukturverbessernden Wirksamkeit

Zur Prüfung der haarkosmetischen Wirkung wurden die erfindungsgemäss zu verwendenden Hexonsäuren und von Disacchariden abgeleiteten On-Säuren in eine wässrige Lösung von 2 Gew.% des Wirkstoffes in entsalztem Wasser überführt.

1.1 Versuchsbeschreibung

Haarsträhnen von ca. 25 cm Länge und ca. 2 g Gewicht der Standard-Type "Light Grey Hair" (Co-de 6626, Fa. Klugmann) wurden auf einer Seite zusammenbegunden und exakt gewogen. Diese Strähnen wurden unter Verwendung einer handelsüblichen Ultrablondiercrème (mit einem Gehalt von 6 Gew.% $H_2O_2$, 10 Gew.-% $(NH_4)_2 S_2O_8$ und einem pH-Wert von 9,0) 45 min lang bei 20°C blondiert. Nach dem Ausspülen mit Leitungswasser von 40°C und Trocknen des Haares im warmen Luftstrom wurden die Strähnen 2 min lang in die 2%ige wässrige Lösung des Strukturant-Wirkstoffes (z.B. Gluconsäure) getaucht. Nach dem Abstreifen der überschüssigen Flüssigkeit wurde die Strähne erneut im warmen Luftstrom getrocknet. Anschliessend wurde die Haarsträhne 30 min lang in eine handelsübliche Dauerwellemulsion (mit einem Gehalt von 9,5 Gew.-% Ammoniumthioglykolat, 5 Gew.-% Ammoniumcarbonat und einem pH-Wert von 8,8) bei 20°C eingelegt und danach mit einer Fixierlösung behandelt. Nach dem Trocknen wurde die Strähne erneut mit der 2%igen Lösung des Strukturant-Wirkstoffes 2 min lang behaldelt und wieder getrocknet.

Dieser Vorgang — Blondierung — Strukturantbehandlung — Dauerwellen — Strukturantbehandlung — wurde dreimal nacheinander durchgeführt, wobei die letzte Strukturantbehandlung entfiel.

Nach dem Trocknen wurden die Haarsträhnen dann unter definierten Bedingungen ausgekämmt. Bei diesem Kämmvorgang trifft, bedingt durch die Haarschädigung, ein Gewichtsverlust auf, der durch Auswiegen bestimmt wurde. Der Blindwert ohne Strukturantbehandlung liegt bei 45 ± 2 Gew.-% Gewichtsverlust.

1.2 Ergebnis

Das Ergebnis der Prüfung für die Strukturant-Wirkstoffe und für Vergleichsprodukte ist der Tabelle zu entnehmen.

| Wirkstoff (2 Gew.-% in Wasser) | Gewichtsverlust (Gew.-%) durch Haarschädigung |
|---|---|
| – | 45±2 Gew.-% |
| Gluconsäure | 24 Gew.-% |
| Lactobionsäure | 28 Gew.-% |
| Glykolsäure | 42 Gew.-% |
| D-Glucuronsäure | 43 Gew.-% |
| D-Zuckersäure | 42 Gew.-% |
| Schleimsäure | 44 Gew.-% |

2. Rezepturbeispiele

Im folgenden werden Rezepturbeispiele für erfindungsgemässe haarkosmetische Mittel zur Verbesserung der Struktur und Widerstandsfähigkeit der Haare angegeben:

2.1 Haarwasser

| | |
|---|---|
| Polyol-Fettsäureester (Cetiol®HE, Henkel) | : 2,0 Gew.-Teile |
| Extrapon® Birke spezial (Dragoco) | : 1,0 Gew.-Teile |
| Lactobionsäure | : 3,0 Gew.-Teile |
| Isopropanol | : 30,0 Gew.-Teile |
| Parfümöl | : 1,0 Gew.-Teile |
| Wasser | : 63,0 Gew.-Teile |

2.2 Haarfestiger

| | |
|---|---|
| Vinylpyrrolidon-Vinylacetat-Copolymer (Luviskol®VA64, BASF) | : 1,5 Gew.-Teile |
| Gluconsäure | : 1,5 Gew.-Teile |
| Isopropanol | : 50,0 Gew.-Teile |
| Parfümöl | : 1,0 Gew.-Teile |
| Wasser | : 46,0 Gew.-Teile |

2.3 Frisiercrème

| | |
|---|---|
| Polyol-Fettsäureester (Cetiol®HE, Henkel) | : 8,0 Gew.-Teile |
| Isopropanol | : 20,0 Gew.-Teile |
| Carbopol®940 (B.F. Goodrich Co.) | : 1,0 Gew.-Teile |
| Triethanolamin | : 0,8 Gew.-Teile |
| Lactobionsäure | : 4,0 Gew.-Teile |
| Parfümöl | : 1,2 Gew.-Teile |
| Wasser | : 65,0 Gew.-Teile |

2.4 Kaltwellemulsion

| | |
|---|---|
| Wasser | : 73,0 Gew.-Teile |
| Gluconsäure | : 2,0 Gew.-Teile |

| | |
|---|---|
| Emulgator (Emulgin®384, Henkel) | : 6,0 Gew.-Teile |
| Komplexbildner (Turpinal®SL, Henkel) | : 0,3 Gew.-Teile |
| Parfümöl | : 0,3 Gew.-Teile |
| Thioglykolsäure | : 8,0 Gew.-Teile |
| Ammoniaklösung, 25%ig in Wasser | : 7,0 Gew.-Teile |
| Ammoniumcarbonat | : 3,0 Gew.-Teile |
| Farbstoffe und Trübungsmittel | : 0,4 Gew.-Teile |

2.5 Kaltwellfixierung

| | |
|---|---|
| Wasser | : 79,0 Gew.-Teile |
| Lactobionsäure | : 2,0 Gew.-Teile |
| Kaliumbromat | : 3,5 Gew.-Teile |
| Fettsäurealkanolamid (Comperlan®KD, Henkel) | : 5,0 Gew.-Teile |
| Natriumlaurylethersulfat 22%ig (Texapon®SG, Henkel) | : 10,0 Gew.-Teile |
| Parfümöl | : 0,3 Gew.-Teile |
| Farbstoff | : 0,2 Gew.-Teile |

2.6 Entwickleremulsion für Färbe- oder Blondiercrème

| | |
|---|---|
| Wasser | : 55,2 Gew.-Teile |
| Ammoniak | : 0,8 Gew.-Teile |
| Gluconsäure | : 2,0 Gew.-Teile |
| Komplexbildner | : 2,0 Gew.-Teile |
| Natriumlaurylethersulfat 28%ig (Texapon®NSO, Henkel) | : 2,0 Gew.-Teile |
| Wasserstoffperoxid-Lösung (50%ig) | : 23,0 Gew.-Teile |
| Polymerisatdispersion (Latekoll®D, BASF) | : 15,0 Gew.-Teile |

**Patentansprüche**

1. Verwendung von On-Säuren, die von Aldohexosen oder Disacchariden mit unverschlossener Aldehydfunktion abgeleitet sind und/oder deren Gamma- oder Delta-Lactonen als Zusätze in Haarwässern, Haarfestigern, Haarsprays, Frisierlotionen, Frisiercremes, Frisiergelen, Wasserwellmitteln, Fönwellmitteln, Schaumtönungsmitteln, Tönungswaschmitteln, Färbemitteln, Dauerwellmitteln, Dauerwellfixierlösungen und Entwickleremulsionen für Oxidationshaarfärbepräparate zur Verbesserung der Struktur und Widerstandsfähigkeit der Haare.

2. Verwendung nach Anspruch 1, dadurch gekennzeichnet, dass als On-Säuren Gluconsäure oder Lactobionsäure und/oder deren γ- oder δ-Lacton verwendet werden.

3. Haarkosmetische Mittel, enthaltend starke Reduktionsmittel, wie die Salze von Mercaptocarbonsäuren oder der schwefligen Säure oder starke Oxidationsmittel, wie Alkalibromat oder Wasserstoffperoxid, gekennzeichnet durch einen Gehalt an On-Säuren, die von Aldohexosen oder Disacchariden mit unverschlossener Aldehydfunktion abgeleitet sind.

4. Haarkosmetische Mittel nach Anspruch 3, gekennzeichnet durch einen Gehalt von 0,5 bis 10 Gew.-% an On-Säuren.

**Revendications**

1. Utilisations d'acides one, qui sont derivés des aldohexoses ou des disaccharides et dont les fonctions aldéhydes ne sont pas bloquées et/ou leurs lactones γ ou δ, comme additifs dans les eaux pour les cheveux, reforçateurs des cheveux, fixateurs, lotions pour la coiffure, crèmes pour la coiffure, gels pour la coiffure, agents ondulants à l'eau produits pour l'ondulation, agents de nuançage moussants, shampooings colorants, teintures, produits pour l'ondulation indéfrisable, solutions destinées à fixer les indéfrisables et émulsions de dévelopement des colorants d'oxydation pour cheveux afin d'améliorer la structure et la résistance des cheveux.

2. Utilisation suivant la revendiation 1, caractérisée en ce que, comme acide-one, on utilise les acides gluconique ou lactobionique et/ou leurs lactones γ ou δ.

3. Produits cosmétiques pour les cheveux contenant un agent réducteur puissant, tel que les sels des acides mercaptocarboxyliques ou des acides sulfureux ou un agent oxydant puissant tel qu'un bromate alcalin ou l'eau oxygénée, caractérisés par une teneur en acides-one qui sont dérivés d'aldohexoses ou de disaccharides dont la fonction aldéhyde n'est pas bloquée.

4. Produits cosmétiques pour les cheveux suivant la revendication 3, caractérisés en ce qu'ils présentent une teneur en acides-one de 0,5 à 10% en poids.

**Claims**

1. The use of onic acids derived from aldohexoses or disaccharides having an unclosed aldehyde function and/or gamma- or delta-lactones thereof as additives in hair lotions, hair lacquers, hair sprays, shaving lotions, shaving creams, shaving gels, water wave preparations, setting preparations, foam tinting preparations, tinting shampoos, dyes, permanent wave preparations, permanent wave setting lotions and developer emulsions for oxidation hair dyeing preparations for improving the structure and resistivity of hair.

2. The use claimed in Claim 1, characterized in that gluconic acid or lactobionic acid and/or the γ- or δ-lactone thereof is used as the onic acid.

3. Trichocosmetic preparations containing strong reducing agents, such as the salts of mercaptocarboxylic acids or sulfurous acid, or strong oxidizing agents, such as alkali bromate or hydrogen peroxide, characterized in that they contain onic acids derived from aldohexoses or disaccharides having an unclosed aldehyde function.

4. Trichocosmetic preparations as claimed in Claim 3, characterized in that they contain from 0.5 to 10% by weight onic acids.